(19) Europäisches Patentamt / European Patent Office / Office européen des brevets

(11) **EP 3 093 781 B1**

(12) **EUROPEAN PATENT SPECIFICATION**

(45) Date of publication and mention of the grant of the patent:
**15.09.2021  Bulletin 2021/37**

(21) Application number: **16020181.0**

(22) Date of filing: **16.05.2016**

(51) Int Cl.:
*G16B 25/00* (2019.01)     *G06F 16/2453* (2019.01)
*G06F 16/2455* (2019.01)    *H03M 13/01* (2006.01)

(54) **SYSTEM AND METHOD FOR TRANSFORMING AND COMPRESSING GENOMICS DATA**

SYSTEM UND VERFAHREN ZUR UMWANDLUNG UND KOMPRIMIERUNG VON GENOMIKDATEN

SYSTÈME ET PROCÉDÉ DE TRANSFORMATION DE LA GÉNOMIQUE ET DE COMPRESSION DE DONNÉES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.05.2015  GB 201508314**

(43) Date of publication of application:
**16.11.2016  Bulletin 2016/46**

(73) Proprietor: **Greenfield, Daniel**
**Cambridge Select CB4 3HP (GB)**

(72) Inventors:
• **Greenfield, Daniel**
  **Cambridge, Select CB4 3HP (GB)**
• **Rrustemi, Alban**
  **Cambridge, CB3 9AX (GB)**

(74) Representative: **Mewburn Ellis LLP**
**Aurora Building**
**Counterslip**
**Bristol BS1 6BX (GB)**

(56) References cited:
• **YU Y WILLIAM ET AL: "Traversing thek-mer Landscape of NGS Read Datasets for Quality Score Sparsification", 2 April 2014 (2014-04-02), CORRECT SYSTEM DESIGN; [LECTURE NOTES IN COMPUTER SCIENCE; LECT.NOTES COMPUTER], SPRINGER INTERNATIONAL PUBLISHING, CHAM, PAGE(S) 385 - 399, XP047268544, ISSN: 0302-9743 ISBN: 978-3-642-36616-1 * abstract * * page 386, paragraph 2 - paragraph 4 ***
• **None**

**Description**

**Technical field**

The present disclosure relates to a computer-implemented method for adjusting the quality score of a genomic sequence base in a genomic sequence read. The method finds uses in compressing information that contains or is related to gene sequences. Furthermore, the present disclosure relates to software products recorded on machine readable data storage media, wherein the software products are executable on computing hardware for implementing aforesaid methods.

**Background**

Yu et al. (Lecture Notes in Computer Science, volume 8394, Research in Computational Molecular Biology, pages 385-399, 2014) describes an approach for compressing read quality scores in sequencing data. The approach sparsifies quality score values by: constructing a database of commonly occurring k-mers throughout a population-sized read data set, identifying k-mers within each read that have a Hamming distance of 1 from the database, discarding any quality scores of read base pairs that match a k-mer and replacing any non-matching quality score above a threshold with a fixed value. This approach does not lead to a score that is interpretable and discards the information in the base call confidence scores.

Therefore, there exists a need for an improved method for transforming quality scores of bases in genomic sequences, in order to improve the quality and compressibility of data.

**Summary**

An approach incorporating insights from Coding Theory is used to boost the signal of bases in High Throughput Sequencing (HTS) outputs according to a conservative prior and Bayesian model. The resultant boosted quality scores are a more accurate representation of the confidence of base calls in each Read, and can be better compressed. Unlike lossy compression schemes that approximate or throw out quality score information, the quality scores from this approach are boosted according to a robust and conservative Coding Theory model. The side-effect is that most quality scores are pushed beyond the saturation point of the Phred scheme resulting in high compression ratios. Importantly, this Bayesian approach does not modify the quality scores of bases unless there is a robust statistical basis for doing so. Instead it improves the underlying quality score of the HTS data under a conservative prior, with improved compression as a side-effect.

In the ordinary case, Coding Theory describes how one can communicate symbols over a noisy communication channel to recover the original symbol. Applying Coding Theory allows one to practically separate out signal from noise utilising a Bayesian approach. The Hamming Distance is used to determine the likelihood that the signal received on the other end corresponds to one symbol versus another. The larger the Hamming Distance between all other symbols, the more likely it is that a symbol can be recovered on the other end and separated from noise. Indeed, the ability of a symbol to tolerate and separate noise grows exponentially with its Hamming Distance to other symbols.

Accordingly, the invention provides a computer-implemented method for adjusting the quality score of a genomic sequence base ($S_j$) in a genomic sequence read (S), the method comprising: performing a search for sequences within a determined or preselected distance of the genomic sequence read (S) in a genomic corpus comprising n-mers (R) from a reference genome for said genomic read, wherein said distance is a Hamming-distance or an edit-distance; based on the results of said search performing calculations of a new quality score for said genomic sequence base ($S_j$) in said read (S) by estimating the probability that the base ($S_j$) in the read (S) has a sequencing error ($\Pr(S_j \neq Z_j \mid S)$) as:

$$\Pr\left(S_j \neq Z_j \mid S\right) \approx \frac{\sum_{k \in L} \Pr\left(S_j \neq Z_j \mid S, R_k\right) \Pr\left(S \mid R_k\right) + \frac{\epsilon_j(3-m)}{3\epsilon_j + 3m - 4m\epsilon_j}\beta}{\sum_{i \in L} \Pr\left(S \mid R_i\right)}$$

where L denotes the set of n-mers ($R_{k \in L}$) within said distance, m denotes a probability of mutation, $e_j$ denotes the probability of a read error at the base ($S_j$) and $\beta$ is an estimate of the contribution of n-mers not within said distance; and replacing the old quality score with the new quality score if the new quality score is better than the old quality score.

In embodiments, the new quality score is constrained to a maximum saturation value. The method may further comprising quantising the new quality score.

Optionally, the calculation in the method utilises the existing quality score of the genomic sequence base that is specified in the genomic sequence read.

Optionally, the method further comprises calculating a new quality score (Q) for the base as $Q=-log10\,P$ where P is the estimated probability that the base ($S_j$) in the read (S) has a sequencing error ($Pr(S_j \neq Z_j \mid S)$).

The method may further comprise determining the search distance. The search distance may be dependent on the expected error rate and the length of the read (S).

Performing a search for sequences within a determined or preselected distance of the genomic sequence read (S) in the genomic corpus may comprise partitioning the genomic sequence read into a multiplicity of slots that each contain a k-mer; for each slot, performing a lookup operation to search for n-mers (R) in the genomic corpus that contain the k-mer of the slot, and combining results from the lookup operation for each said slot to form a list of candidate n-mers (R).

Partitioning the genomic sequence read (S) into a multiplicity of slots that each contain a k-mer may comprise partitioning the genomic sequence read into M+1 slots, where M is a Hamming distance to be used as the determined or preselected distance.

In embodiments, the method includes filtering said list of candidate n-mers (R) so as to exclude those candidate n-mers (R) not within the determined or preselected search distance.

In embodiments, the lookup operation is performed using an index for the reference genome or the genomic corpus, wherein the index is obtained by indexing the reference genome or genomic corpus according to overlapping k-mers.

In embodiments, the slots are not required to have a fixed width.

In embodiments, the index contains a primary index and a secondary index, wherein the primary index is generated from the first bases of each overlapping k-mer, and the secondary index is generated from the remaining bases of each overlapping k-mer. In such embodiments, the method comprises: for said lookup operation, partitioning each slot into a fixed-width primary search key and a non-fixed width secondary search key; performing a primary search by utilising said primary search key in said primary index to obtain a list of candidate n-mers that contain the primary search key as the first bases of a k-mer; and performing a secondary search by utilising said secondary search key in said secondary index to obtain a list of candidate n-mers that is a subset of the list of candidate n-mers, wherein the candidate n-mers in the sublist contain at least a portion of the secondary search key.

In method, within each primary index, the entries are sorted according to the secondary index.. The secondary search can optionally utilise a binary traversal of the sorted entries to find candidate n-mers that contain at least a portion of the secondary search key.

The invention also provides a machine readable data storage medium comprising instructions that, when executed by computing hardware, cause the computing hardware to implement the above methods.

### Description of the diagrams

Embodiments of the present disclosure will now be described, by way of example only, with reference to the following diagrams wherein:

FIG. 1   is a schematic illustration of dividing a symbol search into multiple slot lookup searches
FIG. 2   is a schematic illustration of an individual slot lookup search

### Detailed description of embodiments

As described herein, HTS can be recast and modelled utilising Coding Theory. The transmission model can be based on symbols originating (transmitted) from a corpus (for humans, the corpus may simply be the human reference genome but can be any other representative human genome corpora) that undergoes noise in the form of mutations to form the sample genome, which then undergoes sequencing that introduces further noise in the form of Read errors the result of which is the raw sequencing data (received symbols). In this case the noisy medium has two parts - mutation and sequencing. In the first instance, this approach can ignore indels (insertions and/or deletions - as mutations or as a source of Read errors). For the human genome, the dominant source of variation is due to base changes rather than indels, and the dominant error in Illumina sequencing machines is due to misread bases rather than indels. Thus the type of noise can be considered to primarily be due to base changes, however indels can also be handled in the extended instance.

A Reference Genome (or corpus in general) is considered, which due to mutation with probability $m$ forms a Sample Genome (this could be the genome of a particular individual that is sequenced). From this Sample Genome multiple $n$-mers are randomly constructed which are then sequenced with error probability $\in$ to form a Read. Each of these $n$-mers can be cast back to an equivalent $n$-mer on the Reference Genome. Reference Genome $n$-mers can represent our collection of symbols, and the noisy medium encapsulates the errors introduced at all the stages up to producing the Read. Conservative values are used for each of these stages. The medium has a combined noise probability

of $\mu \equiv m + \epsilon - \frac{4}{3}m\epsilon$. To a first order this is merely the sum of the individual error processes ($m + \epsilon$), but to a second order it can also take into account mutations that have been incorrectly sequenced as the original unmutated version.

One can construct ~3.2 billion symbols of n-mers from the ~3.2 billion bases in the human genome. If these bases were truly random, the average Hamming symbol distance would be expected to be $\frac{3}{4}n$ for any *n*-mer. However, the human genome is most certainly not random. To overcome this, the treatment of nearby symbols is separated from more distant background symbols. Upon transmitting a symbol, the probability that it would arrive as a particular symbol with Hamming Distance *B* is:

$$\mu^B (1 - \mu)^{n-B}$$

Naming Convention:

> $n$ - number of bases in a Read (may vary from Read to Read)
> $S$ - an *n*-mer Read symbol
> $R_k$ - reference *n*-mer symbol $k$
> $G_k$ - true Sample Genome *n*-mer symbol $k$
> $S_j$ - base $j$ of *n*-mer Read
> $\epsilon_j$ - Read error for base $j$ of *n*-mer Read
> $R_{kj}$ - base $j$ of reference symbol $k$
> $G_{kj}$ - base $j$ of true Sample Genome symbol $k$
> $Z_j$ - base $j$ of true Sample Genome source symbol corresponding to the Read

The symbol S corresponds to an *n*-mer Read. $R_k$ is generated from a reference genome by enumerating all possible n-mers from this reference. It is assumed that there is a true Sample Genome that is derived from the reference genome according to a mutation process with per-base probability of mutation *m*. Let $G_k$ be the matching enumeration of all possible *n*-mers from this Sample Genome.

Based on a Markovian mutation process *M* (with probability *m*) and Read error process *E* (with probability $\epsilon$):

$$\Pr(S_j \mid R_{kj}) = \begin{cases} (1 - m)(1 - \epsilon_j) + \frac{1}{3}m\epsilon_j & if\ S_j = R_{kj} \\ (1 - m)\epsilon_j + m(1 - \epsilon_j) + \frac{2}{3}m\epsilon_j & if\ S_j \neq R_{kj} \end{cases}$$

$$\Pr(S \mid R_k) = \prod_j \Pr(S_j \mid R_{kj})$$

The Bayes' theorem gives:

$$\Pr(R_k \mid S) = \frac{\Pr(S \mid R_k)\Pr(R_k)}{\sum_i \Pr(S \mid R_i)\Pr(R_i)}$$

$$= \frac{\Pr(S \mid R_k)}{\sum_i \Pr(S \mid R_i)}$$

With:

$$\Pr(R_k) = \frac{1}{N}$$

for *N* possible *n*-mers, corresponding to uniform sequencing of a genome. Moreover given a particular $R_k$ and *S*, the probability that a base $S_j$ mismatches the source symbol from the Sample Genome corresponding to the Read $Z_j$ (i.e. is a Read error) can be determined according to:

$$\Pr\left(S_j \neq Z_j \mid S, R_k\right) = \begin{cases} \frac{m\epsilon_j}{3-3m-3\epsilon_j+4m\epsilon_j} & if \ S_j = R_{kj} \\ \frac{\epsilon_j(3-m)}{3\epsilon_j+3m-4m\epsilon_j} & if \ S_j \neq R_{kj} \end{cases}$$

Then, removing the dependence on $R_k$, we can determine the Read error per base as:

$$\begin{aligned} \Pr\left(S_j \neq Z_j \mid S\right) &= \sum_k \Pr\left(S_j \neq Z_j \mid S, R_k\right) \Pr\left(R_k \mid S\right) \\ &= \frac{\sum_k \Pr\left(S_j \neq Z_j \mid S, R_k\right) \Pr\left(S \mid R_k\right)}{\sum_i \Pr\left(S \mid R_i\right)} \end{aligned}$$

With ~3.2 billion symbols, this calculation is resource intensive if completed by brute force. By recognising that the contribution of reference symbols decreases exponentially according to their Hamming distance from the Read symbol, this operation can be sped up with negligible error. Let $L$ denote the set of local indices s.t.

$$\forall k \in L, |R_k - S| < B$$

$$\forall k \notin L, |R_k - S| \geq B$$

for some Hamming Distance $B$. The choice of $B$ is ideally such that:

$$\sum_{i \notin L} \Pr\left(S \mid R_i\right) \ll m\epsilon \sum_{i \in L} \Pr\left(S \mid R_i\right)$$

Then:

$$\Pr\left(S_j \neq Z_j \mid S\right) \approx \frac{\sum_{k \in L} \Pr\left(S_j \neq Z_j \mid S, R_k\right) \Pr\left(S \mid R_k\right)}{\sum_{i \in L} \Pr\left(S \mid R_i\right)}$$

There are $3^B \binom{n}{B}$ possible symbols $X_k$ at distance B. To obtain an estimate $\beta$ for the background contribution, the average probability of these symbols $\Pr(S \mid X_k)$ is normalised to $N$ symbols, leading to a value that is typically a very large overestimate and thus conservative in practice:

$$\beta = N\mu^B (1 - \mu)^{n-B}$$

Therefore, a conservative overestimate of each base's Read error can be represented with:

$$\Pr\left(S_j \neq Z_j \mid S\right) \approx \frac{\sum_{k \in L} \Pr\left(S_j \neq Z_j \mid S, R_k\right) \Pr\left(S \mid R_k\right) + \frac{\epsilon_j(3-m)}{3\epsilon_j+3m-4m\epsilon_j}\beta}{\sum_{i \in L} \Pr\left(S \mid R_i\right)}$$

This estimate of a base's Read error represents the new, boosted quality score for the base upon conversion using the Phred scheme:

$$Q = -10 \log_{10} P$$

Where $Q$ is the Phred quality score for an error probability of $P$.
For each Read, the initial distance to search is dependent on the expected error rate and the length of the Read so that longer reads may need larger search distances. For example one may use a search distance based on the worst

case Read error $\varepsilon$ plus some margin multipler (e.g. $2n\,(\varepsilon + m)$). Since the maximum width of a slot may be constrained (say to 32 bases), this may also place a constraint on the minimum distance that can be searched (to in this case $\lceil n/32 \rceil$ - 1). Likewise the background error may be excessive if the search distance is too low, so a minimum distance (e.g. of 5) may also be applied.

Further, the present disclosure is associated with following exemplary methods:

## A) Symbol search

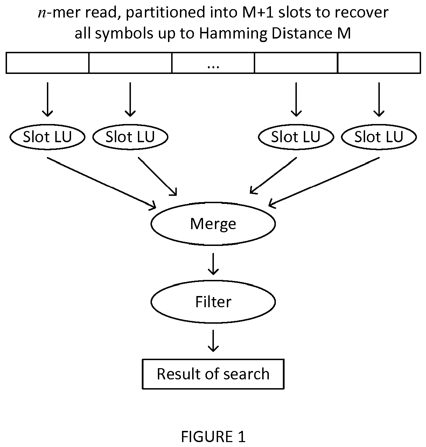

To find all symbols with up to $M$ mismatches from the read, the read is divided into $M + 1$ slots. Based on the Pigeonhole principle, for any symbol up to Hamming distance $M$ away, there must be one slot that does not contain a mismatch (i.e. is an exact match). All slots are searched for all matching symbols. If a particular slot, for example, is a $k$-mer, a search is made to find all symbols that contain that particular $k$-mer. The union of searches across the slots is then guaranteed to contain at least all those symbols within the desired Hamming distance $M$, however it can also contain candidate symbols that are greater than this distance. Filtering is used to discard symbols that are greater than distance $M$. The per-slot search (Slot LU in figure 1) can be achieved by first indexing the reference sequence/corpus according to overlapping $k$-mers as a pre-processing step (e.g. if the corpus contains ACGGCTAC at some position 1004 then a 6-mer index for that would contain position 1004 at index ACGGCT and position 1005 at index CGGCTA and position 1006 at index GGCTAC). For each slot, the set of possible matching symbols is then easily determined by looking up the index for match positions in the corpus. It can be noted that the larger the slot width, the more specific the slot search, and the fewer the possible candidate symbols that need to be examined.

For performance reasons, therefore, it is desirable to have wide slots for searching. However, sometimes more narrow slots are desired (e.g. if searching smaller reads or larger Hamming distances). The following flexible indexing mechanism allows this freedom. For each overlapping $k > 12$ bases, a 24-bit (2-bits per base) primary index is generated from the first 12 bases. For each primary index, the starting position is stored in the reference genome/corpus to the index along with a secondary index of the remaining ($k$ - 12) bases. For example, a secondary index of 8 bits enables 4 additional bases to be stored, resulting in a combined 16 base index, so that a string CTATCGGCTCACTGGA would have a primary index of CTATCGGCTCAC and a secondary index of TGGA. Similarly, a secondary index of 32 bits enables a 28 base index (12 primary+16 secondary). Within each primary index, the entries are sorted according to the secondary index. When searching a slot width of size 12 then, only the primary index is used, and all offsets are retrieved within that index. However when searching a slot width of say size 15, the secondary index is also used to, via binary traversal, retrieve only those offsets that match the additional 3 bases. When searching a slot width that is greater than both the full index size, say of 30 bases when only a 16 base full index is available, then this is achieved by determining the intersection of overlapping 16-base index searches (e.g. a search of CTATCGGCTCACTGGAGCTAACCGATCGAT would consist of a search of CTATCGGCTCACTGGA and GAGCTAACCGATCGAT each represented by slot lookup search, followed by an intersection operation on the results). This methodology allows for rapid and flexible searching of reads within a desired Hamming distance across the reference corpus.

A further speedup can be achieved by making use of the secondary index even when the slot search width is narrow. This is done by directly determining the Hamming distance from the difference between the secondary index and the corresponding section of the Read. If the Hamming distance exceeds the search distance, then we know that this candidate symbol does not meet the search criteria and can be discarded early (rather than at the filtering stage). This saves on random accesses to the reference genome/corpus, resulting in fewer expensive cache misses. In this case, extra bases beyond the slot are also passed to the Slot LU operation (up to a combined size of the full index) to leverage this early filtering operation. For example, a 12-mer slot search of CTATCGGCTCAC where the full index is 20 bases and the Hamming search distance is 3, the slot search operation can be provided the extra 8 bases TGGAGCTA that immediately follow the slot search bases. When determining the list of candidates, instead of adding all items that match the 12-mer search, the secondary index of a candidate can be compared against the extra provided bases to see if it has a Hamming distance of 4 or more, and to thus conditionally exclude candidates.

## B) Read processing

Because a Read may have poor quality scores (high errors) at the head and tail of the Read, a simple preprocessing step involves truncating the Read on either side according to a maximum tolerable quality score (e.g. a probability of Read error of 10% or higher). This truncated result is only used for feeding into the analysis pipeline, and the original Read is not itself truncated.

## C) Quality score quantisation

Boosted quality scores may be improved to such an extent that they represent negligible error. Such quality scores can be constrained to a maximum saturation value $S$ (e.g. 40) beyond which they cannot be further boosted. Those quality scores that are boosted from a value $x$ to a non-saturation value $y < S$ may instead of recording the value $y$, use a quantised value $y' = f(y)$ (e.g. based on the Illumina 8-bin quantisation values) provided $y' > x$. This means that the resultant quality scores are conservatively quantised with the boosting, leading to higher compression ratios.

### D) Edit-distance searches

Reads with indel (base insertion or deletion) variants that are not present in the corpus are likely to result in large Hamming distances to the corpus. This means that it is highly unlikely for such reads to be successfully boosted this way, thus preserving their original quality scores. It is possible to replace Hamming distance searches of a corpus with edit-distance searches instead. An edit-distance search can incorporate a model of both in-place mutations/Read-errors as well as indels. In this case the Markov model from $R_k$ to $G_k$ could incorporate the in-place mutation rate $m_B$ as well as an insertion rate $m_I$ and deletion rate $m_D$ as processes. The sequencing Read error would still be based on the values from the actual Read, however additional estimations of the sequencer insertion error rate $e_I$ and deletion rate $e_D$ could be incorporated as well. Edit-distances searches can be done by again utilising the Pigeonhole principle, but this time accounting for indels as well. Systematic sequencer indels affecting a whole flow-cycle of base reads (such as for Pacific Bioscience sequencers) could also be modelled with a Bayesian approach across affected reads, or by directly incorporating flow-cycle error information into the estimated indel error rates that vary per base across the read.

### E) Long Reads

For long reads (such as those that are thousands of bases long), it may be impractical to process the entire Read at once, so the Read itself could be split into smaller (fixed or variable width) subreads that themselves may benefit from the boosting process. The long Read may then be assembled from the boosted result of these subreads. In some conditions, this can lead to faster and better results than directly applying boosting on the long Read itself. The average amount that quality scores are boosted grows according to the length of the Read being boosted, however this also increases the probability of an indel being encountered and for Hamming-based searches can thus result in little or no boosting. Moreover, there is no point boosting quality scores beyond the saturation threshold. Thus a criteria for determining the split length can be according to both keeping the likelihood of encountering an indel low (<1%) for each subread, as well maintaining a length that ensures most quality scores in the subread are boosted to the saturation threshold. For searches based on edit-distance, the likelihood for encountering indels can be higher and thus longer subreads may still be appropriate, however the likelihood of encountering larger indel regions or of structural variation may still need to be kept low.

Each of the methods A, B, C, D, and E can be used separately or in combination with each other.

### Analysis pipeline

Steps:

1. A pre-processing step of taking a reference genome or other corpus to generate a (dynamic) index
2. For each Read

    2.1. determine a suitable Hamming distance for the search
    2.2. finding all symbols in the corpus within that search distance as candidate source symbols
    2.3. using Bayes for determining the likelihood of candidate source symbols
    2.4. estimating the contribution of all other background source symbols (including assuming zero contribution)
    2.5. using these estimations to calculate a new quality score per base, optionally quantizing based on say worst-case Illumina 8-bin quantisation levels
    2.6. adjusting quality scores based on new calculated quality scores -only if a new quality score is better than the old quality score should one replace the quality score with the new quality score

3. Optionally, to be even more conservative and minimise any possible bias:

    3.1. process all reads against symbol set to find all positions where there are mismatches. Mark these mismatches in the symbol set (e.g. for a reference genome corpus, mark positions in the reference genome that correspond to mismatches)
    3.2. then for each Read, ensure quality scores are preserved at these mismatch positions

**Detailed description of figures**

Referring now to FIG. 1, shown is a schematic illustration of the steps in a search operation of distance up to M. Here an n-mer read is partitioned into M+1 slots, each slot is looked up with a Slot LU operation, the results from which are merged together to form a list of candidate symbols. This is then filtered to include only candidate symbols that are of distance greater than M, which then forms the list of results.

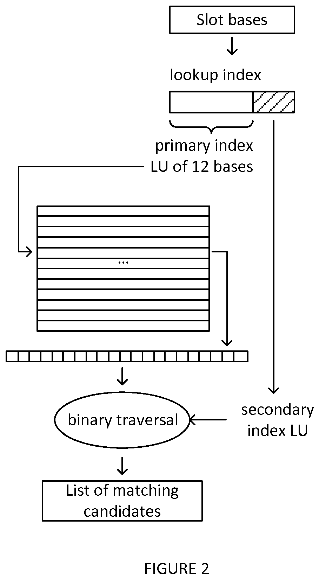

Referring now to FIG. 2, shown is a schematic illustration of the Slot LU operation from FIG 1. Here the input slot bases are partitioned into an input primary index and an input secondary index. The input primary index is used to lookup the primary index table to obtain a list of candidates (each element in the list consists of (i) an offset in the corpus corresponding to candidate symbols, and (ii) secondary index information). The input secondary index is then used to do binary traversal of this list according to its secondary index information, so as to retrieve a subset of the list of candidates that match at least a portion of the input secondary index.

**Claims**

1. A computer-implemented method for adjusting the quality score of a genomic sequence base ($S_j$) in a genomic sequence read (S), the method comprising: performing a search for sequences within a determined or preselected distance of the genomic sequence read (S) in a genomic corpus comprising n-mers (R) from a reference genome for said genomic read, wherein said distance is a Hamming-distance or an edit-distance;

   based on the results of said search performing calculations of a new quality score for said genomic sequence base ($S_j$) in said read (S) by estimating the probability that the base ($S_j$) in the read (S) has a sequencing error ($\Pr(S_j \neq Z_j \mid S)$) as:

$$\Pr\left(S_j \neq Z_j \mid S\right) \approx \frac{\sum_{k \in L} \Pr\left(S_j \neq Z_j \mid S, R_k\right) \Pr\left(S \mid R_k\right) + \frac{\epsilon_j(3-m)}{3\epsilon_j + 3m - 4m\epsilon_j}\beta}{\sum_{i \in L} \Pr\left(S \mid R_i\right)}$$

   where $L$ denotes the set of n-mers ($R_{k \in L}$) within said distance, m denotes a probability of mutation, $e_j$ denotes the probability of a read error at the base ($S_j$) and $\beta$ is an estimate of the contribution of n-mers not within said distance; and

   replacing the old quality score with the new quality score if the new quality score is better than the old quality score.

2. A method as claimed in claim 1, wherein the new quality score is constrained to a maximum saturation value.

3. A method as claimed in claim 1 or claim 2, further comprising quantising the new quality score.

4. A method as claimed in any one of claims 1 to 3, wherein said calculation utilises the existing quality score of the said genomic sequence base in said genomic sequence read.

5. A method as claimed in any one of claims 1 to 4, further comprising calculating a new quality score (Q) for the base as $Q = -log10\ P$ where P is the estimated probability that the base ($S_j$) in the read (S) has a sequencing error ($\Pr(S_j \neq Z_j \mid S)$).

6. A method as claimed in any one of claims 1 to 5, further comprising determining the search distance, wherein the search distance is dependent on the expected error rate and the length of the read (S).

7. A method as claimed in any one of claims 1 to 6, wherein performing a search for sequences within a determined or preselected distance of the genomic sequence read (S) in said genomic corpus comprises:

   partitioning said genomic sequence read (S) into a multiplicity of slots that each contain a k-mer;
   for each slot, performing a lookup operation to search for n-mers (R) in said genomic corpus that contain the k-mer of the slot, and
   combining results from the lookup operation for each said slot to form a list of candidate n-mers (R).

8. A method as claimed in claim 7, wherein partitioning said genomic sequence read (S) into a multiplicity of slots that each contain a k-mer comprises partitioning the genomic sequence read into M+1 slots, where M is a Hamming distance to be used as the determined or preselected distance.

9. A method as claimed in claim 7 or claim 8, wherein said method includes filtering said list of candidate n-mers (R) so as to exclude those candidate n-mers (R) that are not within said determined or preselected distance.

10. A method as claimed in any one of claims 7 to 9, wherein said lookup operation is performed using an index for the reference genome or the genomic corpus, wherein the index is obtained by indexing the reference genome or genomic corpus according to overlapping k-mers.

11. A method as claimed in any one of claims 7 to 10, wherein said slots are not required to have a fixed width.

12. A method as claimed in claim 10 or claim 11, wherein said index contains a primary index and a secondary index, wherein the primary index is generated from the first bases of each overlapping k-mer, and the secondary index is generated from the remaining bases of each overlapping k-mer, the method comprising:

for said lookup operation, partitioning each slot into a fixed-width primary search key and a non-fixed width secondary search key;
performing a primary search by utilising said primary search key in said primary index to obtain a list of candidate n-mers that contain the primary search key as the first bases of a k-mer; and
performing a secondary search by utilising said secondary search key in said secondary index to obtain a list of candidate n-mers that is a subset of the list of candidate n-mers, wherein the candidate n-mers in the sublist contain at least a portion of the secondary search key.

13. A method as claimed in claim 12, wherein within each primary index, the entries are sorted according to the secondary index, and said secondary search utilises a binary traversal of the sorted entries to find candidate n-mers that contain at least a portion of the secondary search key.

14. A machine readable data storage medium comprising instructions that, when executed by computing hardware, cause the computing hardware to implement the method of any one of claims 1 to 13.

**Patentansprüche**

1. Computerimplementiertes Verfahren zum Einstellen der Qualitätsbewertung einer Genomsequenzbase ($S_j$) in einer Genomsequenzauslesung (S), wobei das Verfahren Folgendes umfasst: das Durchführen einer Suche nach Sequenzen innerhalb eines bestimmten oder vorgewählten Abstands der Genomsequenzauslesung (S) in einem Genomkorpus, der n-Mere (R) aus einem Referenzgenom für die Genomauslesung umfasst, wobei der Abstand ein Hamming-Abstand oder ein Edit-Abstand ist;

das Durchführen von auf den Ergebnissen der Suche basierenden Berechnungen einer neuen Qualitätsbewertung für die Genomsequenzbase ($S_j$) in der Auslesung (S) durch Abschätzen der Wahrscheinlichkeit, dass die Base ($S_j$) in der Auslesung (S) einen Sequenzierungsfehler ($Pr(S_j \neq Z_j \mid S)$), wie folgt, aufweist:

$$Pr(S_j \neq Z_j \mid S) \approx \frac{\sum_{k \in L} Pr(S_j \neq Z_j \mid S, R_k) Pr(S \mid R_k) + \frac{\epsilon_j(3-m)}{3\epsilon_j + 3m - 4m\epsilon_j}\beta}{\sum_{i \in L} Pr(S \mid R_i)}$$

worin L für den Satz von n-Meren ($R_{k \in L}$) innerhalb des Abstands steht, m für die Wahrscheinlichkeit einer Mutation steht, $e_j$ für die Wahrscheinlichkeit eines Auslesefehlers an der Base ($S_j$) steht und β für die Abschätzung des Beitrags von n-Meren steht, die nicht innerhalb des Abstands liegen; und
das Ersetzen der alten Qualitätsbewertung durch die neue Qualitätsbewertung, falls die neue Qualitätsbewertung besser ist als die alte Qualitätsbewertung.

2. Verfahren nach Anspruch 1, wobei die neue Qualitätsbewertung auf einen maximalen Sättigungswert beschränkt ist.

3. Verfahren nach Anspruch 1 oder Anspruch 2, das weiters das Quantisieren der neuen Qualitätsbewertung umfasst.

**4.** Verfahren nach einem der Ansprüche 1 bis 3, wobei bei der Berechnung die bestehende Qualitätsbewertung der Genomsequenzbase in der Genomsequenzauslesung verwendet wird.

**5.** Verfahren nach einem der Ansprüche 1 bis 4, das weiters das Berechnen einer neuen Qualitätsbewertung (Q) für die Base mittels $Q = -\log_{10}P$ umfasst, worin P die geschätzte Wahrscheinlichkeit ist, dass die Base ($S_j$) in der Auslesung (S) einen Sequenzierungsfehler ($Pr(S_j \pm Z_j \mid S)))$ aufweist.

**6.** Verfahren nach einem der Ansprüche 1 bis 5, das weiters das Bestimmen des Suchabstands umfasst, wobei der Suchabstand von der erwarteten Fehlerrate und der Länge der Auslesung (S) abhängig ist.

**7.** Verfahren nach einem der Ansprüche 1 bis 6, wobei das Durchführen einer Suche nach Sequenzen innerhalb eines vorbestimmten oder vorgewählten Abstands der Genomsequenzauslesung (S) in dem Genomkorpus Folgendes umfasst:

das Aufteilen der Genomsequenzauslesung (S) in eine Vielzahl von Slots, die jeweils ein k-Mer enthalten;
das Durchführen eines Nachschlagevorgangs für jeden Slot, um nach n-Meren (R) in dem Genomkorpus zu suchen, die das k-Mer des Slots enthalten, und
das Kombinieren der Ergebnisse des Nachschlagevorgangs für jeden Slot, um eine Liste von n-Mer-Kandidaten (R) zu erstellen.

**8.** Verfahren nach Anspruch 7, wobei das Aufteilen der Genomsequenzauslesung (S) in eine Vielzahl von Slots, die jeweils ein k-Mer enthalten, das Aufteilen der Genomsequenzauslesung in M+1 Slots umfasst, wobei M der Hamming-Abstand ist, der als der bestimmte oder vorgewählte Abstand zu verwenden ist.

**9.** Verfahren nach Anspruch 7 oder Anspruch 8, wobei das Verfahren das Filtern der Liste von n-Mer-Kandidaten (R) umfasst, um jene n-Mer-Kandidaten (R) auszuschließen, die nicht innerhalb des bestimmten oder vorgewählten Abstands liegen.

**10.** Verfahren nach einem der Ansprüche 7 bis 9, wobei der Nachschlagevorgang unter Verwendung eines Index für das Referenzgenom oder den Genomkorpus durchgeführt wird, wobei der Index durch Indexieren des Referenzgenoms oder des Genomkorpus nach überlappenden k-Meren erhalten wird.

**11.** Verfahren nach einem der Ansprüche 7 bis 10, wobei die Slots keine fixe Breite aufweisen müssen.

**12.** Verfahren nach Anspruch 10 oder Anspruch 11, wobei der Index einen Primärindex und einen Sekundärindex enthält, wobei der Primärindex aus den ersten Basen jedes überlappenden k-Mers erstellt wird und der Sekundärindex aus den restlichen Basen jedes überlappenden k-Mers erstellt wird, wobei das Verfahren Folgendes umfasst:

das Aufteilen jedes Slots für den Nachschlagevorgang in einen Primärsuchschlüssel mit fixer Breite und einen Sekundärsuchschlüssel ohne fixe Breite;
das Durchführen einer Primärsuche unter Verwendung des Primärsuchschlüssels in dem Primärindex, um eine Liste von n-Mer-Kandidaten zu erhalten, die den Primärsuchschlüssel als erste Basen eines k-Mers enthalten; und
das Durchführen einer Sekundärsuche unter Verwendung des Sekundärsuchschlüssels in dem Sekundärindex, um eine Liste von n-Mer-Kandidaten zu erhalten, die ein Teilsatz der Liste von n-Mer-Kandidaten ist, wobei die n-Mer-Kandidaten in der Teilliste zumindest einen Teil des Sekundärsuchschlüssels enthalten.

**13.** Verfahren nach Anspruch 12, wobei die Einträge innerhalb jedes Primärindex nach dem Sekundärindex sortiert werden und wobei bei der Sekundärsuche ein binäres Traversal der sortierten Einträge verwendet wird, um n-Mer-Kandidaten zu finden, die zumindest einen Teil des Sekundärsuchschlüssels enthalten.

**14.** Maschinenlesbares Datenspeichermedium, das Befehle umfasst, die, wenn sie mittels Computerhardware ausgeführt werden, bewirken, dass die Computerhardware ein Verfahren nach einem der Ansprüche 1 bis 13 durchführt.

**Revendications**

**1.** Procédé mis en œuvre par ordinateur pour ajuster le score de qualité d'une base de séquence génomique ($S_j$) dans

une lecture de séquence génomique (S), le procédé comprenant les étapes consistant à : effectuer une recherche de séquences à l'intérieur d'une distance déterminée ou présélectionnée de la lecture de séquence génomique (S) dans un corpus génomique comprenant des n-mères (R) à partir d'un génome de référence pour ladite lecture génomique, dans lequel ladite distance est une distance de Hamming ou une distance d'édition ;

sur la base des résultats de ladite recherche, effectuer des calculs d'un nouveau score de qualité pour ladite base de séquence génomique ($S_j$) dans ladite lecture (S) en estimant la probabilité que la base ($S_j$) dans la lecture (S) ait une erreur de séquençage (Pr ($S_j{\neq}Z_j$ |S)) sous la forme :

$$\Pr(S_j \neq Z_j \mid S) \approx \frac{\sum_{k \in L} \Pr(S_j \neq Z_j \mid S, R_k)\Pr(S \mid R_k) + \frac{\epsilon_j(3-m)}{3\epsilon_j + 3m - 4m\epsilon_j}\beta}{\sum_{i \in L} \Pr(S \mid R_i)}$$

où $L$ désigne l'ensemble de n-mères ($R_{k \in L}$) à l'intérieur de ladite distance, m désigne une probabilité de mutation, $e_j$ désigne la probabilité d'une erreur de lecture au niveau de la base (Si) et $\beta$ est une estimation de la contribution de n-mères ne se trouvant pas à l'intérieur de ladite distance ; et
remplacer l'ancien score de qualité par le nouveau score de qualité si le nouveau score de qualité est meilleur que l'ancien score de qualité.

2. Procédé selon la revendication 1, dans lequel le nouveau score de qualité est contraint à une valeur de saturation maximale.

3. Procédé selon la revendication 1 ou la revendication 2, comprenant en outre une quantification du nouveau score de qualité.

4. Procédé selon l'une quelconque des revendications 1 à 3, dans lequel ledit calcul utilise le score de qualité existant de ladite base de séquence génomique dans ladite lecture de séquence génomique.

5. Procédé selon l'une quelconque des revendications 1 à 4, comprenant en outre le calcul d'un nouveau score de qualité ($Q$) pour la base sous la forme $Q=-log_{10}P$, où P est la probabilité estimée que la base ($S_j$) dans la lecture (S) ait une erreur de séquençage (Pr($S_j{\neq}Z_j$|S))

6. Procédé selon l'une quelconque des revendications 1 à 5, comprenant en outre la détermination de la distance de recherche, dans lequel la distance de recherche dépend du taux d'erreur attendu et de la longueur de la lecture (S).

7. Procédé selon l'une quelconque des revendications 1 à 6, dans lequel la réalisation d'une recherche de séquences à l'intérieur d'une distance déterminée ou présélectionnée de la lecture de séquence génomique (S) dans ledit corpus génomique comprend les étapes consistant à :

partitionner ladite lecture de séquence génomique (S) en une multiplicité de fentes qui contiennent chacune un k-mère ;
pour chaque fente, effectuer une opération de consultation pour rechercher des n-mères (R) dans ledit corpus génomique qui contiennent le k-mère de la fente, et
combiner des résultats de l'opération de consultation pour chaque dite fente pour former une liste de n-mères candidats (R).

8. Procédé selon la revendication 7, dans lequel la partition de ladite séquence génomique (S) en une multiplicité de fentes qui contiennent chacune un k-mère comprend la partition de la lecture de séquence génomique en M+1 fentes, où M est une distance de Hamming à utiliser en tant que distance déterminée ou présélectionnée.

9. Procédé selon la revendication 7 ou la revendication 8, dans lequel ledit procédé comprend le filtrage de ladite liste de n-mères candidats (R) de manière à exclure les n-mères candidats (R) qui ne sont pas dans ladite distance déterminée ou présélectionnée.

10. Procédé selon l'une quelconque des revendications 7 à 9, dans lequel ladite opération de consultation est effectuée en utilisant un index pour le génome de référence ou le corpus génomique, dans lequel l'index est obtenu en indexant le génome de référence ou le corpus génomique selon des k-mères en chevauchement.

**11.** Procédé selon l'une quelconque des revendications 7 à 10, dans lequel lesdites fentes ne sont pas nécessaires pour avoir une largeur fixe.

**12.** Procédé selon la revendication 10 ou la revendication 11, dans lequel ledit index contient un index primaire et un index secondaire, dans lequel l'index primaire est généré à partir des premières bases de chaque k-mer se chevauchant, et l'index secondaire est généré à partir des bases restantes de chaque k-mère en chevauchement, le procédé comprenant les étapes consistant à :

pour ladite opération de consultation, partitionner chaque fente en une clé de recherche primaire à largeur fixe et une clé de recherche secondaire à largeur non fixe ;
effectuer une recherche primaire en utilisant ladite clé de recherche primaire dans ledit index primaire pour obtenir une liste de n-mères candidats qui contiennent la clé de recherche primaire en tant que premières bases d'un k-mère ; et
effectuer une recherche secondaire en utilisant ladite clé de recherche secondaire dans ledit index secondaire pour obtenir une liste de n-mères candidats qui est un sous-ensemble de la liste de n-mères candidats, dans lequel les n-mères candidats dans la sous-liste contiennent au moins une partie de la clé de recherche secondaire.

**13.** Procédé selon la revendication 12, dans lequel, dans chaque index primaire, les entrées sont triées selon l'index secondaire, et ladite recherche secondaire utilise une traversée binaire des entrées triées pour trouver des n-mères candidats qui contiennent au moins une partie de la clé de recherche secondaire.

**14.** Support de stockage de données lisible par machine comprenant des instructions qui, lorsqu'elles sont exécutées par du matériel informatique, amènent le matériel informatique à mettre en œuvre le procédé de l'une quelconque des revendications 1 à 13.

*n*-mer read, partitioned into M+1 slots to recover
all symbols up to Hamming Distance M

| | | ... | | |
|---|---|---|---|---|

Slot LU   Slot LU        Slot LU   Slot LU

Merge

Filter

Result of search

FIGURE 1

Slot bases

↓

lookup index

primary index
LU of 12 bases

...

secondary
index LU

binary traversal

List of matching
candidates

FIGURE 2

**EP 3 093 781 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **YU et al.** *Lecture Notes in Computer Science, volume 8394, Research in Computational Molecular Biology,* 2014, vol. 8394, 385-399